# EUROPEAN PATENT SPECIFICATION

(11) **EP 0 725 605 B1**
(45) Date of publication and mention of the grant of the patent: **07.07.1999**
(21) Application number: 94915894.3
(22) Date of filing: 26.04.1994
(51) Int. Cl.: A61F 2/30, A61F 2/36, A61L 27/00, B29C 45/00, B28B 19/00

(54) **PROSTHESIS WITH INTEGRAL PROXIMAL SPACER**
PROTHESE MIT EINGEBAUTEN PROXIMALEN DISTANZHALTERN
PROTHESE A PIECE D'ESPACEMENT PROXIMALE SOLIDAIRE

(30) Priority: 26.10.1993 US 14589
(43) Date of publication of application: 14.08.1996
(73) Proprietor: HOWMEDICA INC., New York New York 10017 (US)
(72) Inventor: KASHUBA, Glen, River Edge, NJ 07661 (US); MICHIELLI, Michael, Hoboken, NJ 07030 (US); GOLDENBERG, J., Mel, Upper Saddle River, NJ 07458 (US); KLIPPEL, Jon, I., Basking Ridge, NJ 07920 (US)
(74) Representative: Haug, Dietmar, Dipl.-Ing.
(86) International application number: US9404570
(87) International publication number: WO9511640

(56) References cited:
- EP-A- 0 149 527
- EP-A- 0 321 389
- EP-A- 0 385 585
- EP-A- 0 501 116
- WO-A-93/01770
- US-A- 4 612 160
- US-A- 4 795 472
- US-A- 4 881 536
- US-A- 5 047 054
- US-A- 5 089 004

## Description

### RELATED APPLICATIONS

The present application is a continuation-in-part of application serial no. 29/018,452, filed February 14, 1994, which is a continuation of application serial no. 29/014,589, filed October 26, 1993, now abandoned.

### BACKGROUND OF THE INVENTION

The present invention relates to orthopaedic implants in general, and more particularly to a prosthesis used in conjunction with a cement spacer.

In hip replacement surgery, an enlarged canal is created through the reaming and broaching of the proximal femur and the subsequent removal of bone tissue. Bone cement is then inserted into the canal using standard methodology. Following the insertion of the bone cement, the implant must be inserted into the canal in a timely fashion with the proper alignment, before the bone cement cures. It is desirable to provide a prosthesis that can be easily implanted in the correct anatomical position and will therefore form a strong bond within a bone canal with the supplied bone cement.

It is known to provide implants with a coating of material to help bonding with bone cement in a bone canal. For example, in U.S. Patent No. 4,283,799 to Pratt, Jr. et al., a pre-coated hip prosthesis is disclosed. All or most of the surface of the prosthesis is covered with a bone cement material that is bonded to the prosthesis. Similarly, U.S. Patent No. 4,491,987 to Park discloses a prosthesis with a uniform polymer coating on substantially the entire surface of the prosthesis. In addition, U.S. Patent Nos. 4,281,420 and 4,336,618 to Raab provide a prosthesis coated with a polymethylmethacrylate (PMMA) film fixedly adhered to the surface. The film is applied to the prosthetic surface after treating, and then the film is annealed. Similarly, U.S. Patent No. 4,365,359 to Raab utilizes a silane coupling agent to adhere the PMMA film to a prosthetic element and further enhance the adherence of the prosthesis. EP-A-0 501 116 discloses a prosthetic implant for insertion into a cement filled intramedullary canal including a plurality of spacers formed from PMMA. The spacers include a body having a leading portion and a trailing portion. The body of the spacers narrowing proximally from said leading portion toward said trailing portion. A solid coating of bone compatible polymer around the proximal portion of a prosthesis is disclosed in U.S. Patent No. 4,881,536 to Noble et al. The coating forms an integral collar of a desired thickness and shape. The collar thickness serves to operably effect a seal with the bone. The tapered shape of the collar thickness assures bone cement flow into all bone crevices and minimizes bone cement porosity by reducing the size of the voids formed by entrapped air during curing of the bone cement.

Another technique to increase the adherence of the prosthesis to the bone cement is the use of a textured surface on the prosthesis. An example of such a surface is shown in U.S. Patent No. 4,795,472 to Crowninshield et al.

While the pre-coated and textured surfaces described above may assist a prosthesis in better adhering to the bone cement and bone, they do not provide certain other beneficial aspects of total hip replacement surgery including pressurization of the bone cement as the prosthesis is inserted and alignment of the prosthesis centrally within the bone canal.

Separate spacers for centralizing the prosthesis in the bone canal are disclosed in the art, for example in U.S. Patent No. 4,827,919 to Barbarito et al. In use, such spacers are placed over the proximal opening of the bone canal either immediately before or after the bone cement is injected into the canal. The prosthesis is then inserted through the spacer into the canal. Such spacers have the disadvantage that, as the prosthesis is inserted, the bone cement is forced out of the canal and obscures the surgeon's view of the spacer, such that it is difficult to determine whether the prosthesis is properly seated. The spacer also has a slight tendency to lift up from the bone site due to the pressure of the expelled bone cement.

### SUMMARY OF THE INVENTION

An object of the invention is to provide a prosthesis with an integral proximal spacer that allows for improved bonding, repeatable accuracy of placement of the prosthesis in a patient, and easier insertion of the prosthesis into the femoral canal.

An additional object of the invention is to provide a prosthesis with an integral proximal spacer that allows for improved pressurizing and aligning of the prosthesis within the bone cement in a bone canal. The prosthesis of the present invention is specifically directed for use in hip replacement surgery, with the prosthesis consisting of a hip femoral component that is inserted into the femur of a patient after removing the top portion and a central area of the femur. It is understood, however, that the concept of the present invention could be adapted for use in other orthopaedic surgery, such as knee surgery.

These and other objects are achieved according to the present invention by a combined prosthesis and integral, proximal spacer preferably made of a bone cement material molded onto the prosthesis. The spacer is shaped to pressurize the additional bone cement within the enlarged canal upon insertion, and to align the prosthesis centrally within the canal. In order to ensure a secure bond between the spacer and prosthesis shaft, a grit-blasted, textured surface is preferably provided on the prosthesis. For further bonding improvement, a series of dimples can be provided on the surface of the prosthesis. Also, a textured surface may be provided on the prosthesis adjacent the spacer for increased adherence to bone cement in the canal.

The integral bone cement spacer according to the present invention is preferably made by an injection molding process. The process includes the use of high pressure injection molding, using powdered and/or pelletized PMMA under heat and pressure to form the spacer around the prosthesis.

To prepare for the molding process, the prosthesis first undergoes a gas plasma cleaning process that prepares the surface of the prosthesis prior to the molding of the bone cement spacer. In this process, an energized oxygen and argon gas mixture removes any surface contaminants from the prosthesis and leaves the substrate surface "active", i.e. highly pure and devoid of contaminants. The process provides an extremely clean, contaminate-free surface for the prosthesis. The active surface ensures optimal bonding between the substrate and the molded PMMA. The prosthesis is then preheated via an induction heating process, prior to the high pressure molding process.

### BRIEF DESCRIPTION OF THE DRAWINGS

FIG. 1 is a perspective view of a prosthesis according to the invention illustrating the base element and integral spacer;
FIG. 2 is an medial view of the prosthesis of FIG. 1;
FIG. 3 is an lateral view of the prosthesis of FIG. 1;
FIG. 4 is a cross-sectional view of the prosthesis through line A-A of FIG. 1;
FIG. 5 is a cross-sectional view of the prosthesis through line B-B of FIG. 1;
FIG. 6 is a side view of an alternative spacer attached to the prosthesis;
FIG. 7 is a cross-sectional view of the prosthesis through line D-D in FIG. 6;
FIG. 8 is an enlarged cross-sectional view through line C-C in FIG. 3, showing a typical interlocking dimple;
FIG. 9 is a perspective view of a bone canal broach sizing the femoral canal for insertion of the prosthesis of the invention.

### DETAILED DESCRIPTION

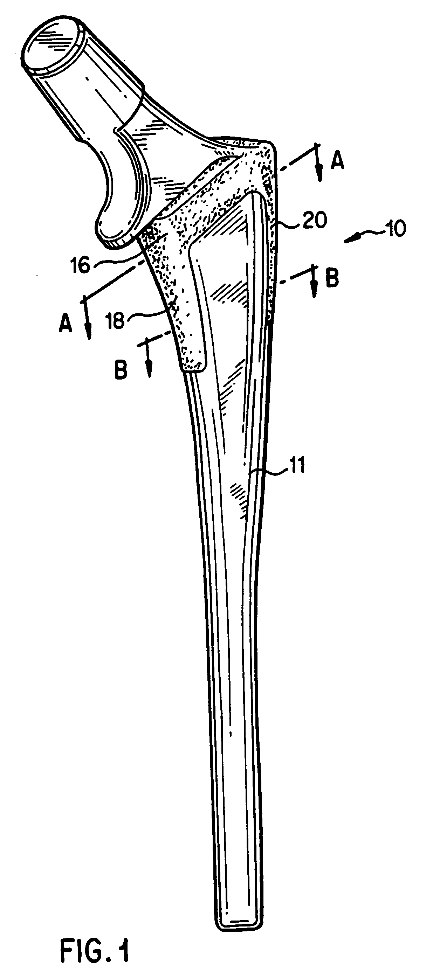

FIG. 1 illustrates a femoral prosthesis 10 comprising a prosthesis base element or shaft 11 with an integral proximal spacer 12 according to the present invention. The general shape of a femoral prosthesis is known and disclosed, for example, in U.S. Patent No. 4,881,536 to Noble et al. The prosthesis base element is preferably constructed of a cobalt-chrome alloy, although other alloys are acceptable including stainless steel and titanium alloys.

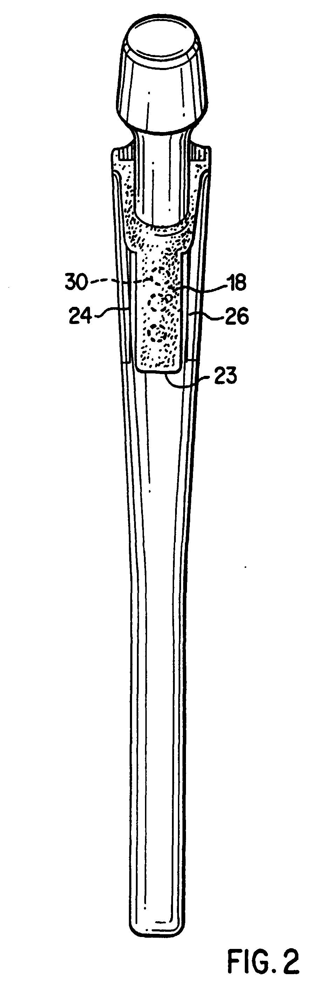

Spacer 12 is illustrated in FIG. 1 as it appears affixed to the base element 11. The spacer has a circumferential portion 16 and, in this embodiment, three distally extending legs 18, 20, and 22. First leg 18 extends distally along the medial face of the prosthesis 10, as best shown in FIG 2.

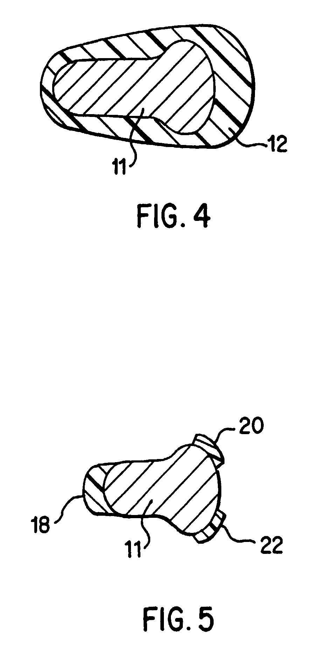

The second leg 20 and third leg 22 extend distally along the lateral-anterior and lateral-posterior corners, as shown in FIG. 3. The legs preferably range in length from 1.524 cm to 2.8575 cm (.600'' to 1.125''). Legs 18, 20, and 22 each have a gradually tapering shape ending in a feathered or tapered edge 23, which blends smoothly against the prosthesis substantially without discontinuity so as to create a surface that is inserted into a bone canal with little resistance and without catching on the edge of the canal. The configuration of the legs also prevents the formation of any voids or discontinuities within the additional cement supplied for implantation. The tapering is preferably approximately five degrees. Spacer 12 generally aids the surgeon in the correct alignment of the prosthesis within the bone canal, as the tapered legs 18, 20, and 22 allow for a gliding insertion, and the circumferential portion 16 assures final positioning. The cross-sectional views are shown in FIGS. 4 and 5.

To ensure correct alignment, the canal is preferably prepared, as shown in FIG. 11, with a bone canal broach 60 matched to the size of the prosthesis and integral spacer. The broach 60 and spacer are match-sized so as to create a substantially controlled distance between the circumferential portion 16 of the spacer and the bone canal opening 62. With the canal properly sized to match the spacer, circumferential portion 16 acts to pressurize the bone cement during the final insertion of the prosthesis and spacer into the bone canal.

Spacer 12 thus endures neutral alignment of the prosthesis in the bone canal and the circumferential portion 16 helps to pressurize the bone cement within the canal. Excessive pressurization, however, is to be avoided to prevent blowout of the distal cement bone plug and difficulty in final implant seating. This problem is prevented in the present invention by the provision of the separate legs, preferably three legs 18, 20, and 22, which create spaces for cement "venting" as the prosthesis is introduced into the bone canal. This also allows the newly introduced cement to distribute evenly in the femoral canal. Final pressurization occurs only when circumferential portion 16 enters the canal and prevents further venting of cement. The spacer's tripod shape also allows for repeatable molding manufacture, and allows for reduced potential for voids or shrink to be present in the final molded article.

The unique shape of the bone cement spacer 12 provides other advantages for the implantation of the prosthesis. The ⁵tripod" structure of relatively narrow circumferential portion 16 and three extending legs 18, 20, and 22 provides relief zones 24, 26, and 28 between the molded arms of the geometry, allowing for "gradual" alignment and pressurization upon insertion into the canal. This graduation assures a minimization of voids or discontinuities within the additional cement supplied to the bone canal for implantation. The circumferential portion is approximately .508 cm - .762 cm (.2-.3 inches) wide in the preferred embodiment, and the legs are approximately .762 cm to 1.27 cm (.3-.5 inches) wide.

The prosthesis 10 is preferably provided with two areas off texture 14, one on the anterior side and one on the posterior side of base element 11 as shown in FIGS. 9 and 10. These areas are not covered by the spacer. The textured 14 areas are designed to provide very strong mechanical interlock at the locations where the prosthesis will be under significant amounts of torque rotation and stress between the prosthesis and bone cement. The texture can be in the form of a waffled macro-texture as shown by reference numeral 14, or, e.g., vertical grooves. The remaining surface of base element 11 has a slightly rough texture as is known in the art (such as formed by grit blasting).

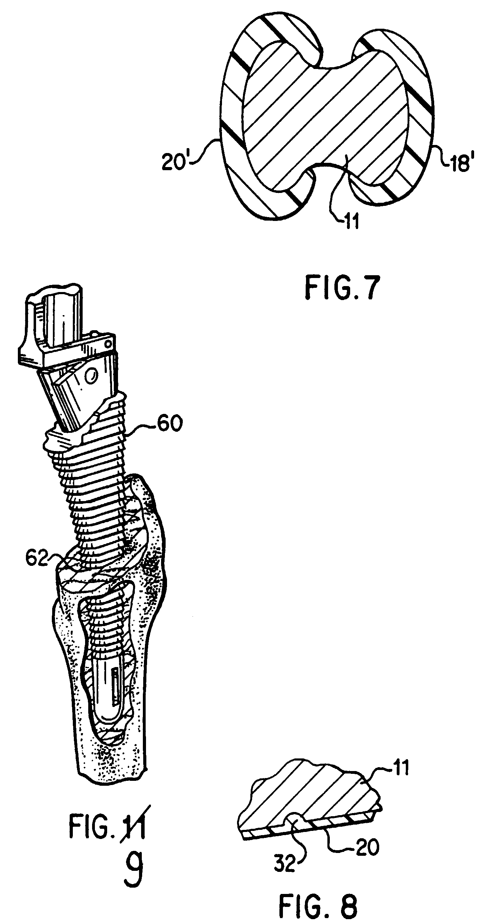

The prosthesis is further preferably provided with a mechanical interlock between the spacer and base element. Base element 11 has a plurality of dimples, or concave hemispheres, located on the surface of the base element on the lateral and medial sides under the spacer. Referring to FIGS. 2 and 3, most preferably, there are three dimples 30 on the medial side, and six (two rows of three) dimples 32 on the lateral side. A cross-section of a typical dimple is illustrated in FIG. 8. A mechanical interlock is formed when the spacer is molded around the base element and flows into the dimples. The interlock is potentially strengthened by the shrink properties of the PMMA material used for the spacer as the PMMA cures onto the prosthesis. The key attributes of the dimples which maximize interlock are the location of the dimples, their depth, radius and angulation with respect to the stem surface.

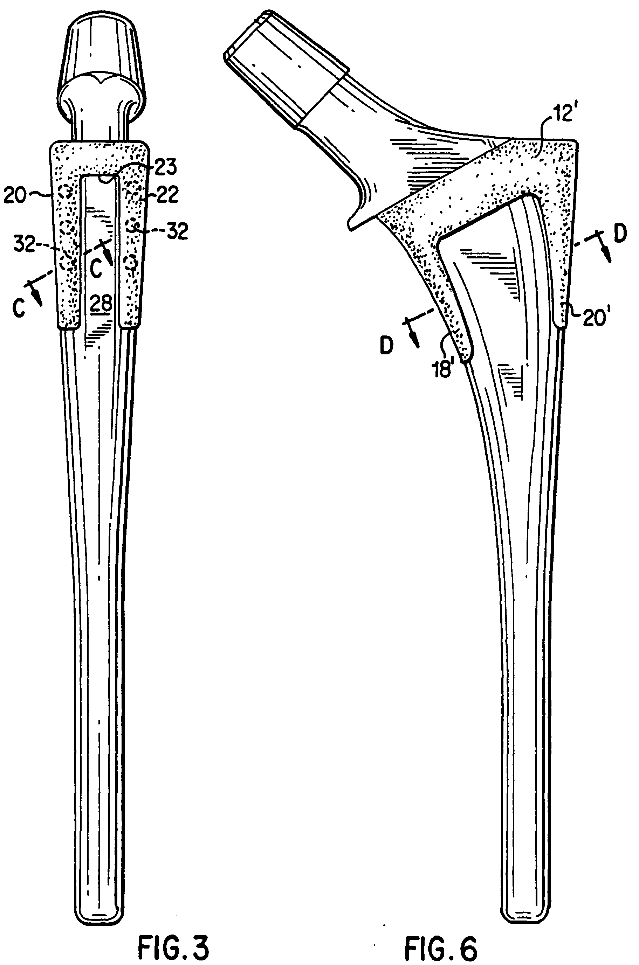

FIGS. 6 and 7 illustrate an alternative embodiment in which only two extending legs are utilized. According to this alternative embodiment, spacer 12' is provided with only two distally extending legs: medial leg 18' and lateral leg 20'. FIG. 7 shows how the two distally extending legs 18', 20' of the alternative embodiment encompass the prosthesis. Spacer 12' is otherwise substantially the same as spacer 12.

In each of the embodiments, the spacer is preferably injection molded directly onto the base element, to provide an integral, single piece prosthesis for implantation and to ensure a strong bond between the base element and the spacer. The manufacturing process involves preparing the base element for the molding, and then molding the spacer thereon. To prepare the base element for molding, it is subjected to a gas plasma cleaning process. Preferably, a Gas Plasma Unit Model C-24 from Advanced Plasma Systems, Inc. is utilized. The gas plasma unit applies an ionized mixture of oxygen and argon gases (40% O₂, and 60% Ar) to the prosthesis, which provides a clean, active, oxidized surface that is extremely wettable and allows the molded material to adhere to the prosthesis easily.

In use or the gas plasma unit, the prosthesis is placed on a fixture to hold the element, and placed in a chamber. The chamber is then closed, and evacuated to 299.925 pascals (225 millitorr). The gas mixture of 40% O₂ and 60% Ar is then added. The plasma field is energized by a 950 watt radio frequency (R.F.). Fresh gases are circulated through the chamber at a rate of .5 standard liters per minute (S.L.M.). After twenty minutes of R.F., the chamber is purged with nitrogen gas.

The prosthesis and the supporting fixture is then placed in an induction heater and preheated to about 260°C +/- 28°C (500°F +/- 50°F), and then placed directly into the mold, such that the prosthesis does not drop significantly in temperature. Preheating the prosthesis before the molding step enhances the cement to substrate transition at the critical end points of the molded geometry. The raised temperature of the prosthesis provides a neutral environment for the PMMA attachment and for uniform cooling of the prosthesis and spacer.

The spacer is preferably molded from commercial grade PMMA (which may be in pelletized/granular form or a finer powdered form) blended with about 7% by weight BaSO₄ powder that has been extruded into pellets. The mold itself is modular to fit all stem sizes. The outer portion of the mold is made of stainless steel, and the inner portion is a flexible elastomeric liner that prevents the prosthesis from being scratched or deformed and allows spacers of different dimensions to be molded under high pressure. The elastomeric liner also allows for the formation of the geometry of the 4 legs of the spacer. Those geometries would be difficult to create using conventional stainless stool molds. In the molding process, the raw material is loaded into a sealed hopper, and the mold is preheated to 49°C +/-6°C (120°F +/- 10°F). The preheated prosthesis is placed into the preheated mold cavity, and the mold is closed and the molding cycle begins. The initial pressure is built up to 10.342 MPa (1500 psi), and the mold is filled to a certain volume with the raw material. The pressure is reduced to 1.724 MPa (250 psi) until the complete fill of the mold is achieved. The pressure is hold until the mold cavity pressure starts to decrease. The pressure drops to zero, and the clamp pressure is maintained until the part solidifies, and the prosthesis can be ejected. The molded spacer is then trimmed as necessary with a hot knife.

For final finishing, the prosthesis is inspected visually using X-rays, and sterilized with gamma irradiation.

As an alternative, molding can be accomplished it lower temperature and pressure by first mixing the bone cement powder with a curing agent and infecting the liquid bone cement into the mold to harden.

## Claims

1. A prosthesis (10) comprising:
an elongated base element (11) with a proximal end and a distal end for insertion into a bone canal; and
a spacer member (12) secured to the base element with a circumferential portion (16) completely surrounding the proximal end of said base element, characterized in that said spacer member (12) further having at least two separate legs (18,20), each leg extending distally along the base element (11), said legs tapering to a reduced thickness and width in the distal direction.

2. The prosthesis (10) of claim 1 wherein said legs are tapered to provide feathered outer edges to facilitate insertion.

3. The prosthesis (10) of claim 1 wherein said circumferential portion (16) has a tapered distal edge (23) to facilitate insertion into the bone canal.

4. The prosthesis (10) of claim 1 wherein said circumferential portion (16) defines at least one vent space to allow escape of bone cement as the prosthesis is inserted into the bone canal to prevent excessive pressurization of bone cement in the canal.

5. The prosthesis (10) of claim 1 wherein the base element (11) defines at least one depression therein disposed under the spacer member (12) such that a part of said spacer member (12) extends into the depression to create a mechanical interlock therebetween.

6. The prosthesis (10) of claim 5 wherein said base element (11) defines a plurality of said depressions with at least one said depression disposed under each leg of said spacer member (12).

7. The prosthesls (10) according to claim 1, wherein:
said base element (11) is configured and dimensioned for insertion into a femoral bone canal as a shaft of a femoral prosthesis; and
said at least two legs comprise a first leg (18) extending distally along the medial face of the prosthesis shaft and at least a second leg (20) extending distally along the lateral face of the prosthesis shaft.

8. The prosthesis (10) of claim 7 wherein said at least a second leg (20) comprises the second leg extending distally along the lateral-anterior surface of the shaft and a third leg (22) extending distally along the lateral-posterior surface of the shaft wherein said second and third legs terminate at spaced apart distal ends.

9. The prosthesis (10) of claim 8 wherein the base element (11) defines a plurality of depressions (30) therein with at least one said depression disposed under each leg of the spacer member such that a part of each leg extends into said depressions to create a mechanical interlock therebetween.

10. The prosthesis (10) of claim 9, wherein there are at least three said depressions arranged in a row under each said leg.

11. The prosthesis (10) of claim 1 wherein the spacer (12) is molded directly onto the prosthesis from PMMA.

12. The prosthesis (10) claim 7 further comprising a plurality of concave hemispheres (30) formed in the medial and lateral aides of said base element (11), said hemispheres receiving protrusions of said spacer (12) to create an improved bond between the spacer and the base element.

13. The prosthesis (10) of claim 7 further comprising an area of textured surface (14) over a portion of the base element (11) between said legs.

14. The prosthesis (10) of claim 1 wherein said base element (11) in constructed of a titanium alloy.

15. A prosthesis implantation kit, comprising the prosthesis of claim 1 in combination with a bone canal reamer, wherein said circumferential portion of the prosthesis spacer member defines an outer profile and said reamer defines an outer reaming profile configured and dimensioned to ream the bone canal to substantially match said circumferential portion outer profile for sealing contact between the bone canal and spacer member such that bone cement deposited in the canal is pressurized when the prosthesis is inserted therein and seated against the reamed canal.

16. A process of producing a prosthesis according to claim 1 comprising the steps of:
providing a metallic base element (11) for the prosthesis;
subjecting said element (11) to gas plasma cleaning in an oxygen and argon rich environment;
heating said base element (11) to an elevated temperature of about 260°C (500°F), such that the surface of the base element (11) is clean and wettable; and
molding a bone cement spacer (12) around at least a portion of said element, after the heating step, said spacer (12) having a circumferential portion (16) completely surrounding the proximal end of said base element (11) and further having at least two separate legs (18,20), each leg extending distally along the base element (11), said legs tapering to a reduced thickness and width in the distal direction.

17. The process of claim 16 further comprising a step of grit blasting said base element before the gas plasma cleaning step to provide a textured surface for enhanced adherence of bone cement.

18. The process of claim 16 wherein the gas plasma cleaning process comprises the steps of:
placing said base element (11) in a chamber;
evacuating said chamber of air;
adding a gas mixture of oxygen and argon to said chamber;
ionizing the gas mixture; and circulating fresh gases in said chamber.

19. The process of claim 16, wherein said molding comprises:
placing the bane element (11) in a mold substantially immediately after said heating such that the temperature of the base element (11) does not substantially reduce prior to molding;
injecting dry powdered PMMA into said mold to form said spacer (12).

## Patentansprüche

1. Prothese (10), die folgendes aufweist:
- ein längliches Grundelement (11) mit einem proximalen Ende und einem distalen Ende zur Einführung in einen Knochenkanal und
- ein Spacerelement (12), das an das Grundelement mit einem ringförmigen Teil (16) befestigt ist, welches das proximale Ende des Grundelements vollständig umgibt, dadurch gekennzeichnet, daß das Spacerelement (12) außerdem mindestens zwei getrennte Beine (18,20) aufweist, die sich jeweils distal entlang des Grundelements (11) erstrecken, wobei die Beine sich in der distalen Richtung auf eine geringere Dicke und Breite verjüngen.

2. Prothese (10) nach Anspruch 1, wobei die Beine sich verjüngen, so daß sich scharfe äußere Kanten zur erleichterten Einführung ergeben.

3. Prothese (10) nach Anspruch 1, wobei der ringförmige Teil (16) eine sich verjüngende distale Kante (23) zur erleichterten Einführung in den Knochenkanal aufweist.

4. Prothese (10) nach Anspruch 1, wobei der ringförmige Teil (16) mindestens eine Auslaßöffnung definiert, durch die der Knochenzement austreten kann, wenn die Prothese in den Knochenkanal eingeführt wird, so daß eine übermäßige Druckbeaufschlagung des Knochenzements in dem Kanal vermieden wird.

5. Prothese (10) nach Anspruch 1, wobei das Grundelement (11) mindestens eine Vertiefung darin definiert, die unter dem Spacerelement (12) angeordnet ist, so daß sich ein Teil des Spacerelements (12) in die Vertiefung erstreckt und dazwischen eine mechanische Verriegelung erzeugt wird.

6. Prothese (10) nach Anspruch 5, wobei das Grundelement (11) eine Vielzahl von Vertiefungen definiert und mindestens eine der Vertiefungen unter jedem Bein des Spacerelements (12) angeordnet ist.

7. Prothese (10) nach Anspruch 1, wobei:
- das Grundelement (11) zur Einführung als Schaft einer Femurprothese in einen femoralen Knochenkanal ausgestaltet und dimensioniert ist und
- die mindestens zwei Beine ein erstes Bein (18), das sich distal entlang der mittleren Oberfläche des Prothesenschafts erstreckt, und mindestens ein zweites Bein (20), das sich distal entlang der seitlichen Oberfläche des Prothesenschafts erstreckt, umfassen.

8. Prothese (10) nach Anspruch (7), wobei das mindestens zweite Bein (20) das zweite Bein, das sich distal entlang der seitlichen vorderen Oberfläche des Schafts erstreckt, und ein drittes Bein (22), das sich distal entlang der seitlichen hinteren Oberfläche des Schafts erstreckt, umfaßt, wobei das zweite und das dritte Bein in voneinander beabstandeten distalen Enden enden.

9. Prothese (10) nach Anspruch 8, wobei das Grundlement (11) eine Vielzahl von Vertiefungen (30) darin definiert, von denen mindestens eine der Vertiefungen unter jedem Bein des Spacerelements so angeordnet ist, daß ein Teil jedes Beins sich in die Vertiefungen erstreckt, so daß dazwischen eine mechanische Verriegelung erzeugt wird.

10. Prothese (10) nach Anspruch 9, wobei mindestents drei der Vertiefungen in einer Reihe unter jedem Bein angeordnet sind.

11. Prothese (10) nach Ansruch 1, wobei der Spacer (12) direkt auf der Prothese aus PMMA geformt wird.

12. Prothese (10) nach Anspruch 7, die außerdem eine Vielzahl von konkaven Halbkugeln (30) aufweist, die in den mittleren und seitlichen Seiten des Grundelements (11) gebildet sind, wobei die Halbkugeln Protrusionen des Spacers (12) aufnehmen, so daß eine verbesserte Verbindung zwischen dem Spacer und dem Grundelement erzeugt wird.

13. Prothese (10) nach Anspruch 7, die außerdem eine Fläche mit texturierter Oberfläche (14) auf einem Teil des Grundelements (11) zwischen den Beinen aufweist.

14. Prothese (10) nach Anspruch 1, wobei das Grundelement (11) aus einer Titanlegierung konstruiert ist.

15. Prothesenimplantationskit, der die Prothese nach Anspruch 1 in Kombination mit einem Knochenkanalbohrer aufweist, wobei der ringförmige Teil des Prothesenspacerelements ein äußeres Profil definiert und der Bohrer ein äußeres Bohrerprofil definiert, das so ausgestaltet und dimensioniert ist, das der Knochenkanal so gebohrt wird, das er weitgehend mit dem äußeren Profil des ringförmigen Teils zusammenpaßt, so daß der Knochenkanal durch das Spacer-element so verschlossen wird, daß der sich in dem Kanal befindende Knochenzement mit Druck beaufschlagt wird, wenn die Prothese darin eingeführt und in den gebohrten Kanal eingepaßt wird.

16. Verfahren zur Herstellung einer Prothese nach Anspruch 1, bei dem:
- ein Metallgrundelement (11) für die Prothese bereitgestellt wird,
- das Element (11) der Gasplasmareinigung in einer sauerstoff- und argonreichen Umgebung unterzogen wird;
- das Grundelement (11) auf eine erhöhte Temperatur von etwa 260°C (500°F) erhitzt wird, so daß die Oberfläche des Grundelements (11) sauber und benetzbar wird, und
- nach dem Erhitzungsschritt ein Knochenzementspacer (12) um mindestens einen Teil des Elements geformt wird, wobei der Spacer (12) einen ringförmigen Teil (16), der das proximale Ende des Grundelements (11) vollständig umgibt, und außerdem mindestens zwei getrennte Beine (18,20) aufweist, die sich jeweils distal entlang des Grundelements (11) erstrecken, wobei die Beine sich in der distalen Richtung auf eine geringere Dicke und Breite verjüngen.

17. Verfahren nach Anspruch 16, wobei das Grundelement außerdem vor dem Gasplasmareinigungsschritt zur Bereitstellung einer texturierten Oberfläche zur verbesserten Haftung von Knochenzement sandgestrahlt wird.

18. Verfahren nach Anspruch 16, wobei das Gasplasmareinigungsverfahren die folgenden Schritte umfaßt:
- das Grundelement (11) wird in einer Kammer angeordnet wird;
- aus der Kammer wird die Luft durch Evakuieren entfernt;
- ein Gasgemisch aus Sauerstoff und Argon wird in die Kammer gegeben;
- das Gasgemisch wird ionisiert und
- frische Gase werden in die Kammer zirkuliert.

19. Verfahren nach Anspruch 16, wobei die Formgebung folgendes umfaßt:
- das Grundelement (11) wird praktisch sofort nach dem Erhitzen in ein Formwerkzeug gegeben, so daß die Temperatur des Grundelements (11) vor der Formgebung praktisch nicht abnimmt,
- trockenes, gepulvertes PMMA wird in das Formwerkzeug eingespritzt, um den Spacer (12) zu bilden.

## Revendications

1. Prothèse (10), comprenant :
un élément allongé de base (11) ayant une extrémité proximale et une extrémité distale destinée à être introduite dans un canal d'un os, et
un organe d'entretoise (12) fixé à l'élément de base avec une partie circonférentielle (16) qui entoure totalement l'extrémité proximale de l'élément de base, caractérisée en ce que l'organe d'entretoise (12) possède en outre au moins deux branches séparées (18, 20), chaque branche s'étendant du côté distal le long de l'élément de base (11), les branches ayant une dimension qui diminue vers une épaisseur et une largeur réduite, en direction distale.

2. Prothèse (10) selon la revendication 1, dans laquelle les branches sont effilées afin qu'elles donnent des bords externes amincis destinés à faciliter l'insertion.

3. Prothèse (10) selon la revendication 1, dans laquelle la partie circonférentielle (16) a un bord distal incliné (23) qui facilite l'insertion dans le canal osseux.

4. Prothèse (10) selon la revendication 1, dans laquelle la partie circonférentielle (16) délimite au moins un espace de ventilation destiné à permettre l'échappement d'un ciment osseux lorsque la prothèse est insérée dans le canal de l'os pour empêcher une mise sous pression excessive du ciment osseux dans le canal.

5. Prothèse (10) selon la revendication 1, dans laquelle l'élément de base (11) délimite au moins une cavité disposée sous l'organe d'entretoise (12) afin qu'une partie de l'organe d'entretoise (12) s'étende dans la cavité pour créer un emboîtement mécanique entre elles.

6. Prothèse (10) selon la revendication 5, dans laquelle l'élément de base (11) délimite plusieurs cavités avec au moins une cavité placée sous chaque branche de l'organe d'entretoise (12).

7. Prothèse (10) selon la revendication 1, dans laquelle :
l'élément de base (11) a une configuration et des dimensions permettant l'insertion dans le canal de l'os fémoral sous forme d'une tige de prothèse fémorale, et
les deux branches au moins comprennent une première branche (18) qui s'étend du côté distal le long de la face médiale de la tige de la prothèse et au moins une seconde branche (20) qui s'étend du côté distal le long de la face latérale de la tige de la prothèse.

8. Prothèse (10) selon la revendication 7, dans laquelle une seconde branche au moins (20) forme la seconde branche qui s'étend du côté distal le long de la surface latérale-antérieure de la tige et une troisième branche (22) qui s'étend du côté distal le long de la surface latérale-postérieure de la tige, si bien que la seconde et le troisième branche se terminent à des extrémités distales espacées.

9. Prothèse (10) selon la revendication 8, dans laquelle l'élément de base (11) délimite plusieurs cavités (30) ayant au moins une cavité placée sous chaque branche de l'organe d'entretoise, de manière qu'une partie de chaque branche s'étende dans les cavités pour créer un emboîtement mécanique entre elles.

10. Prothèse (10) selon la revendication 9, dans laquelle trois cavités au moins sont placées en ligne sous chaque branche.

11. Prothèse (10) selon la revendication 1, dans laquelle l'entretoise (12) est directement moulée sur la prothèse à partir de PMMA.

12. Prothèse (10) selon la revendication 7, comprenant en outre plusieurs hémisphères concaves (30) formées aux côtés médial et latéral de l'élément de base (11), les hémisphères logeant des saillies de l'entretoise (12) pour la création d'une excellente liaison entre l'entretoise et l'élément de base.

13. Prothèse (10) selon la revendication 7, comprenant en outre une région de surface texturée (14) placée sur une partie de l'élément de base (11) entre les branches.

14. Prothèse (10) selon la revendication 1, dans laquelle l'élément de base (11) est construit à partir d'un alliage de titane.

15. Kit d'implantation de prothèse, comprenant la prothèse selon la revendication 1 en combinaison avec un alésoir pour canal osseux, dans lequel la partie circonférentielle de l'organe d'entretoise de la prothèse délimite un profil externe et l'alésoir délimite un profil externe d'alésage dont la configuration et les dimensions sont telles qu'il assure l'alésage du canal osseux afin que celui-ci corresponde pratiquement au profil externe de la partie circonférentielle pour assurer un contact étanche entre le canal osseux et l'organe d'entretoise, si bien que le ciment déposé dans le canal est mis sous pression lorsque la prothèse est insérée et est mise en appui contre le canal alésé.

16. Procédé de production d'une prothèse selon la revendication 1, comprenant les étapes suivantes :
la disposition d'un élément métallique de base (11) pour la prothèse,
le traitement de l'élément (11) par nettoyage par un plasma gazeux dans un milieu riche en oxygène et en argon,
le chauffage de l'élément de base (11) à une température élevée d'environ 260 °C (500 °F) afin que la surface de l'élément de base (11) soit propre et mouillable, et
le moulage d'une entretoise (12) de ciment osseux autour d'une partie au moins de l'élément, après l'étape de chauffage, l'entretoise (12) ayant une partie circonférentielle (16) qui entoure totalement l'extrémité proximale de l'élément de base (19) et ayant en outre au moins deux branches séparées (18, 20), chaque branche s' étendant du côté distal le long de l'élément de base (11), les branches ayant une dimension qui varie vers une épaisseur et une largeur réduite dans la direction distale.

17. Procédé selon la revendication 16, comprenant en outre une étape de sablage de l'élément de base avant l'étape de nettoyage par un plasma gazeux pour la formation d'une surface texturée destinée à augmenter l'adhérence du ciment osseux.

18. Procédé selon la revendication 16, dans lequel l'opération de nettoyage par un plasma gazeux comprend les étapes suivantes :
la disposition de l'élément de base (11) dans une chambre,
l'évacuation de l'air de la chambre,
l'addition d'un mélange gazeux d'oxygène et d'argon dans la chambre,
l'ionisation du mélange gazeux, et
la circulation de gaz neufs dans la chambre.

19. Procédé selon la revendication 16, dans lequel le moulage comprend :
la disposition de l'élément de base (11) dans un moule pratiquement juste après le chauffage afin que la température de l'élément de base (11) ne diminue notablement avant le moulage, et
l'injection de PMMA en poudre sèche dans le moule pour la formation de l'entretoise (12).
